(19) Europäisches Patentamt
European Patent Office
Office européen des brevets

(11) **EP 4 290 219 A1**

(12) **EUROPEAN PATENT APPLICATION**

(43) Date of publication:
**13.12.2023   Bulletin 2023/50**

(51) International Patent Classification (IPC):
***G01N 21/359*** (2014.01)

(21) Application number: **22178046.3**

(52) Cooperative Patent Classification (CPC):
**G01N 21/359; G01N 33/225;** G01N 2201/129

(22) Date of filing: **09.06.2022**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Lietuvos Agrariniu Ir Misku Mokslu
Centras
58344 Akademijos mstl., Kédainiu r. (LT)**

(72) Inventors:
• **Slepetiene, Alvyra
Dotnuvos sen. Kedainiu r. (LT)**
• **Parasotas, Irmantas
Kedainiai (LT)**

• **Veteikis, Darijus
Saldenes km., Vilniaus raj. sav. (LT)**
• **Ceseviciene, Jurgita
Akademija, Kedainiu r. (LT)**
• **Amaleviciute-Volunge, Kristina
Kedainiai (LT)**
• **Mankeviciene, Audrone
Akademija, Dotnuvos sen. Kedainiu r. (LT)**
• **Skersiene, Aida
Dotnuvos sen. Kedainiu r. (LT)**
• **Volungevicius, Jonas
Vilnius (LT)**

(74) Representative: **Klimaitiene, Otilija
AAA Law
A. Gostauto 40B
03163 Vilnius (LT)**

(54) **DETERMINATION OF THE COMPOSITION OF BIOGAS PRODUCTION BY-PRODUCT USING A NEAR-INFRARED SPECTROSCOPY METHOD**

(57)    A method for estimating the chemical composition of solid and liquid biogas-production anaerobic digestates by near-infrared spectroscopy, comprising:

- providing weighted anaerobic digestate samples (2a, 2b);

- performing near-infrared spectroscopy analysis (4) on the provided weighted samples (2a, 2b), to obtain near-infrared spectroscopy spectra (5) thereof;

- applying for each obtained spectrum (5) regression equations (6) comprising, at least, calibrated coefficients of determination between near-infrared spectroscopy spectra and known chemical analytes to estimate the concentration of said known chemical analytes in the chemical composition (7) of the weighted anaerobic digestate samples (2a, 2b).

The method quickly and accurately determinates the amounts of ash, organic carbon and other elements important for plant nutrition and soil improvement

Fig. 1

EP 4 290 219 A1

**Description**

TECHNICAL FIELD

The invention relates to the field of agricultural biotechnology, more specifically, to methods for digestate composition determination. It discloses a method for determining the composition of digestate, by employing near-infrared spectroscopy (NIRS) measurements and subsequent processing of the measured NIRS data.

BACKGROUND ART

Anaerobic digestate (AD) is a biogas production by-product. It is important to assess the potential benefits of the digestates (which may be available in solid and liquid states) as the biofertilizers, and as the improvers of the soil properties. For determination of its value, it is necessary to determine the chemical composition of this waste biomass. This purpose requires fast and efficient methods of analysis. Various analytical methods are used for this purpose. Among which, we do not detect the application of Near-infrared spectroscopy (NIRS) for determination of chemical composition of different states of the digestate.

This invention is aimed to improve the quality of diagnostics of digestate, a by-product of biogas production. The method was developed to evaluate specific chemical composition of digestate which could be used to predict its chemical composition variation and possible effects on the soils.

NIR spectroscopy is a powerful tool for qualitative and quantitative phytoanalysis. It is a rapid and high-throughput analytical method, with on-site capability, high chemical specificity, and requiring no or minimal sample preparation. NIR spectroscopy is a non-invasive and low-cost alternative with significant practical advantages compared to the conventional methods of analysis (Bec, 2021; Mallet, 2021; Ozaki, 2018; Galvez-Sola, 2010; Albrecht, 2009; Huang, 2008).

Several studies of compost or organic wastes using NIRS have shown that NIRS could be an effective estimation method. For example, until now NIRS technology has been used in similar areas to determinate physico-chemical parameters and nutrient metal contents in animal manure compost (Albrecht, 2009; Huang, 2008; Malley, 2005); carbon and nitrogen contents in sewage sludge (Galvez-Sola, 2013); green waste compost (Zhen, 2016; Albrecht, 2011; Albrecht, 2008); nitrogen content in poultry manure compost (Galvez-Sola, 2010; Fujiwara and Murakami, 2007); physico-chemical parameters, organic matter, total organic carbon and concentrations of different nitrogen forms and cell wall fractions of sewage sludge compost (Zhang, 2017; Vergnoux, 2009); heavy metal contents in sewage sludge (Galvez-Sola, 2013; Moral, 2007) and compost (Albrecht, 2011; Galvez-Sola, 2010; 2009a); nitrogen content in sewage sludge (Gálvez-Sola, 2010), humic acid from sewage sludge (Albrecht, 2011; Polak, 2005); nutrient composition of layer manure (Xing, 2008) and fattening pig manure (Beccaccia, 2015; Yang, 2006); organic waste (Mallet, 2021).

Very little is known about the use of the NIRS for the management and the traceability of the composting process of agroindustrial composts (Galvez-Sola, 2010).

NIRS is an instrumental method of spectroscopy that uses the near-infrared range of wavelengths (from 780 nm to 2500 nm) of the electromagnetic spectrum. Typical NIRS applications include medical and physiological diagnostics, pharmaceutical, food and agrochemical quality control, atmospheric chemistry and other. Methods of qualitative and quantitative chemical analyses are time-consuming and uneconomical in terms of the amounts of chemical reagents used which are relatively costly. One of the ways to optimise analyses time, volume and costs is the application of non-destructive (non-invasive), with minimal or no sample preparation, real-time, accurate, chemical-free near-infrared reflectance spectroscopy (NIRS) measurements safe for the measured object, laboratory staff and the environment. It is a multi-analytical technique: several determinations can be made simultaneously. Once calibrated, the NIRS spectrometer is a device, simple to use and operate. Instrumentation for NIRS is like instruments for the UV-visible and mid-IR ranges. There is a source, a detector, and a dispersive element (such as a prism or a diffraction grating) to allow the intensity at different wavelengths to be recorded. The introduction of NIRS into the analytical world as an accepted technique began in the 1950s. But just after the introduction of light-fiber optics in the mid-1980s and the monochromator-detector developments in the early 1990s, NIRS became a more powerful tool for scientific research.

Non-invasive NIRS method utilize reflectance signals resulting from bending and stretching vibrations in molecular bonds between carbon, nitrogen, hydrogen, and oxygen. Absorption bands relating to many chemical bonds, such as C-H, N-H, O-H, C=O, C=C etc. in the NIR region are typically very broad and overlap, which means that the conventional univariate calibration techniques using only one wavelength per component for evaluations cannot be applied in the cases of overlapping bands. The usual way of employing NIR spectroscopic data to predict the analyte values is to construct a multivariate calibration model. In a form of regression equations such models are often employed to extract the desired chemical information, which can be used to predict the composition of unknown samples from NIR measurements. This means that it is necessary to relate optical data to the information of reference analysis obtained in a chemical laboratory. Therefore, careful development of a set of calibration samples and application of multivariate calibration techniques is essential for near-infrared analytical methods (Murray, 2001; Givens et al, 1997).

Equipment of NIRS at the Lithuanian Research Centre for Agriculture and Forestry, Chemical Research laboratory until now was applicable for the determination of the composition of forage grasses and legumes and its mixtures, maize, different types of silage, wheat grain, rapeseed, and soil. The fodder grasses, maize plants, silage have been evaluated for crude protein, crude fat, water soluble carbohydrates (WSC), starch, crude fibre, neutral detergent fibre (NDF), acid detergent fibre (ADF) and crude ash. Was accumulated optical and chemical data base developed from high to good precision calibration equations for wheat quality indicators - grain moisture, protein, and starch contents. NIRS calibrations also were developed for soil quality components displaying from good to very good accuracy in the prediction of the humus, C organic, C Dumas, N Dumas, fulvic and humic acids and N Kjeldahl.

Patent literature describes inventions in the field of digestates and similar organic materials and methods applying NIRS analyses.

The US Patent document US 9,678,002 B2 relates to the use of NIR spectroscopy for the analysis of chemical compositions, and more particularly, for the near real time analysis of components during 10 production of hydrocarbons. However, the teaching of US 9,678,002 B2 cannot be applied to determine such important indicators for valorization and practical use of digestates as in this invention (N, P, K, Mg, Ca, Cu, Fe, Zn, acid detergent fibre (ADF), neutral detergent fibre (NDF), organic matter (OM), dry matter (DM), humic acids, organic carbon).

The US Patent US20050010374A1 describes the method of analysis of NIRS data. A method for providing qualitative analysis of solid forms of a chemical compound/or drug candidate including polymorphous, hydrates, solvates and amorphous solids, that does not require a prior knowledge of either the solid form or the total number of groups of solid forms.

Patent application WO2014060687A2 describes digestate treatment method, fertilizer resulting from the said method, and corresponding treatment unit. The invention relates to the method and the unit for treating a digestate extracted from a methanization plant digestion tank. According to the invention, such a method includes: a step for mixing at least the liquid fraction of said digestate with ana essentially inorganic material that contains a substantial amount of clay, zeolite, and/or diatomaceous earth, for a duration long enough to allow said material to at least partially trap ionized ammonia nitrogen contained in said digestate; and a step for separating the solid fraction from said mixture.

One more Patent document EP 1 199 555 describes an analytical method and apparatus for liquid samples using near infrared spectroscopy. This invention relates to a NIR spectroscopy method for liquid samples.

Patent application document WO 2004/042375 describes NIR spectroscopy method for analysing chemical process components. This invention relates to a NIR spectroscopy method for analysis of chemical process components, e.g., for analysis of bitumen and solvent-diluted bitumen from froth treatment during oil sands processing.

The closest prior art is the international patent application WO 2020/099657. This document discloses the process for processing biomass digestate for obtaining, e.g., biogas, lignin and nutriens like phosphorus and nitrogen. The method comprises the steps of providing a biomass digestae subjecting the biomass digestate to separation into liquid fraction and a solid fraction, and obtaining post-treated biomass digestate. Both inventions previously described and the present invention, are related to the studies of digestates and their possible applications, which can vary by method and effectiveness. However, in our case, an effective method is presented to determine the value of digestates. It includes many new indicators such as organic matter (OM), organic carbon (OC), humic acids, ash, elements, etc., not only phosphorus (P) and nitrogen (N). According to the approved patent, a description of the method of separation of digestate into a liquid fraction and a solid digestate fraction is provided. Our invention differs from the one described in that for the development of an analytical near infrared spectroscopy method, fractions of liquid and solid digestate obtained in real time during the biogas production process were studied, while the process of digestate formation itself is out of scope of this research.

The above reviewed patent documents , still do not satisfy a need for a faster and more effective method for compositional analysis of ADs. The method, disclosed in the present document, is developed to improve the existing by-product analysing methods and is intended to solve the technical problem for faster and more precise non-invasive analysis of anaerobic digestates under industrial conditions.

SUMMARY OF INVENTION

With the development of a new type of energy sources and alternatives, as the number of biogas production plants increases, huge quantities of this production waste (anaerobic digestate) are generated. The quality of digestates may be very different, it changes over time, therefore, to for using the digestate as a bio-fertilizer, it is necessary to have effective methods for determining its quality.

To solve this problem, the purpose of the invention is to assess the ability of near infrared spectroscopy (NIRS) to determine chemical properties of an anaerobic digestate. The invention elaborates an innovative, fast, and accurate method using the NIR spectroscopy for assessing the digestate chemical composition. This invention relates to a NIRS method for analyses of digestates provided in solid and liquid states as a by-product of biogas production.

This NIRS method is developed and described to enable digestate analysis to be carried out by an effective, non-destructive method. Also, the method achieves such a beneficial effect that no chemical reagents and substances are used for analysis. One more of the advantages of the method is that it is safe for the environment. The advantageous effect of the invention is also that with this method it is possible to analyze digestates of different states, liquid and solid. Using this NIR spectroscopic method, it is possible quickly and at a low cost to determine the chemical composition of digestates, which determines its use as a bio-fertilizer and soil improver. There are many indicators that show the quality of digestates, which has not been described until now. The mathematical relationships between the NIRS spectra and the chemical composition parameters of the digestates have already been obtained in preparation for the patent application. This is expected to provide a more accurate prediction of the impact of bioproducts on crop yields, soil and environment.

BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:

Fig. 1 depicts analyzing process by NIRS device for the sample of the digestate: 1a - Solid digestate sample; 2a - drying and milling; 3a - Small ring cup; 1b - Liquid digestate sample; 2b - HDPE bag (encapsulating into HDPE bag); 4 - NIRS analysis by NIR-6500 Perstorp Analytical; 5 - NIRS Spectrum; 6 - NIRS Spectrum analysis equations; 7 - Chemical composition of digestate.

Fig. 2 depicts parameters of calibration of NIRS data of solid digestate for ash determination in solid digestate. Number of Samples 21; RSQ 0.813; Bias 0.645; SEP 1.375

Fig. 3 depicts parameters of calibration of NIRS data of solid digestate for organic matter (OM) determination in solid digestate. Number of Samples 21; RSQ 0.807; Bias 0.258; SEP 1.404

Fig. 4 depicts parameters of calibration of NIRS data of liquid digestate for dry matter (DM). Number of Samples 16; RSQ 0.941; Bias -0.202; SEP 3.326

Fig. 5 depicts parameters of calibration of NIRS data of liquid digestate for organic matter (OM). Number of Samples 16; RSQ 0.628; Bias 1.726; SEP 2.606

Preferred embodiments of the invention are described herein below with reference to the drawings. Each figure contains the same numbering for the same or equivalent element.

DETAILED DESCRIPTION OF THE INVENTION

The present invention advantageously relates to developing an analytical method. The essence of the invention consists in the development of the NIRS method to determine the chemical composition of liquid and solid states of digestate by scanning samples with the NIRS 6500 device using equations suitable for AD analysis. The instrument NIRS-6500 was equipped with WINISI III v1.50e (®Infrasoft International, LLC) software and SPINNING and TRANSPORT modules.

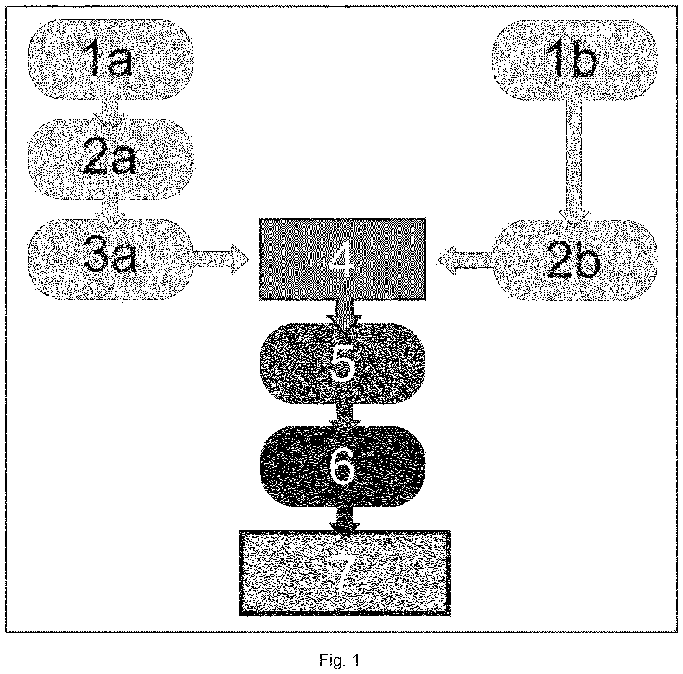

The scheme of the analysis process is shown in Fig. 1.

*Sampling of anaerobic digestate (AD).*

Samples of AD by-product digestate recovered from the agricultural waste were removed from the reactor and both output streams (solid and liquid states). About 5 kg of separated solid digestate and 5 L of liquid digestate were collected and delivered to the laboratory. All samples were thoroughly mixed before being dispensed into 0.25 L plastic bottles and stored at -20 °C until analysis. Industrial biogas power plants of the same production capacity, located in Lithuania, were selected for the digestate sampling. The chemical composition of the samples of solid fraction and liquid fraction of digestate from the 8 biogas plants in the country was determined. The calibration set of AD samples was composed of different samples collected in 2018-2021. To develop the method and to create calibration equations, 106 samples of solid digestates and 107 samples of liquid digestates from 9 biogas plants were used.

*AD samples scanning principles.*

AD spectra of the reflection from the bonds (log 1 / R) are recorded at 2 nm intervals. All samples were scanned in three replications.

*AD samples scanning procedure. Solid digestate.*

Samples were oven-dried at 65 ± 5 °C temperature to a constant weight and grounded in an ultra-centrifugal mill ZM 200 (Retsch, Germany) to pass a 1 mm screen. All the samples of the solid state of the AD samples were scanned on a monochromator NIR Systems model 6500 *(Perstorp Analytical, USA)* equipped with SPINNING module using a small ring cup (4.7 cm).

*AD sample scanning procedure. Liquid digestate.*

All the samples of the liquid state of the AD were scanned on a monochromator NIR Systems model 6500 *(Perstorp Analytical, USA)* equipped with TRANSPORT module rectangular cell with internal dimensions of 4.1 cm wide, 17.2 cm long diameter (54.12 cm$^2$ sample surface area). The liquid sample is poured into a clear sample bag (High Density Polyethylene (HDPE), Whirl-pak® liquid sample bags), 120 ml bag (3/4 full) corresponding to the size of the cell of the TRANSPORT module. This method of scanning in bags of liquid AD samples was tested and advantageously developed for the first time, and we did not detect such descriptions in the literature.

*NIRS spectra calibration.*

Calibration requires two prerequisite stages. The first stage is the collection of analytical values from the samples analysed by reference methods. For each analysis of solid and liquid digestate components were used a large group of samples analysed using standard laboratory analytical procedures and using results as reference data to receive quantitative calibration for NIRS spectroscopy.

The measurements of the NIR spectra, is the second stage.

Calibration itself is essentially a complex statistical analysis aiming to find the optimal equations predicting concentrations of different chemical compounds in samples. An equation that relates log 1/R (log of 1/reflectance) values at selected key wave lengths to chemistry values enables a calibration to be developed provided sufficient samples have been scanned to represent the full range of constituents. Calibration is achieved by collecting spectra from a large population of samples typical of those to be tested in future. It is important to update calibrations with new samples added to represent a wide range of environmental conditions. This requirement was confirmed previously by Givens et al., 2017; Murray, 2001. It is important to include AD samples with great variety in their chemical composition. Accurate NIRS prediction systems depend upon availability of a large calibration database, accuracy of reference methods used for calibration.

Different spectral pre-processing and regression methods like partial least squares (PLS) and modified partial least squares (Modified PLS) were advantageously explored to enhance the relation between the spectra and measured digestate properties.

A further advantage of the invention is, that this invention offers an effective, fast determination of chemical properties of anaerobic digestate obtained as a by-product of biogas production. Once the calibration is achieved with satisfactory equation statistics (R$^2$, Standard Error of Prediction), the set of equations can be further employed for the detection of AD composition. In this way, the invention offers a technological chain that consists of obtaining the spectral data for the digestate using NIRS spectrometer and processing it with and adequate software, like WINISI III v1.50e (®Infrasoft International, LLC) applying a certain set of equations written as a single file. The equation file contains a set of indexes acquired through analysis involving the test of a great variety of optical data scatter correction and mathematical treatment techniques.

A further advantage of the invention, is that in order to develop the method to determine the composition of liquid and dry digestate, equations from spectrometric data were selected from nearly 200,000 regression analysis calculations combining a variety of optical data scatter correction and mathematical treatment techniques applying WINISI III v1.50e (®Infrasoft International, LLC) software. Equations predicting values of 21 analytes of liquid digestate and 18 analytes of dry digestate went through calibration, cross-validation, and testing (prediction with new data).

The advantage of the invention is that the content of dry matter (RSQ = 0.943) in liquid digestate and the content of ash (0.813), organic carbon (0.807), magnesium (0.788), potassium (0.724) and copper (0.711) in solid digestate are best predicted. The equations for the other analytes have prediction RSQ < 0.7. Knowledge of spectral data correction and mathematical treatment techniques as well as equation indexes is an essential part of the described method.

***Reference methods used to determine the chemical composition of two states* of anaerobic digestate**

Reference chemical methods have been used to determine the composition of anaerobic digestate (AD).

*Nitrogen determination in solid digestate.* The Kjeldahl method was used to determine the nitrogen in solid AD. Weighing approx. 0.2 g of the dried homogeneous sample. Placing the sample into a digestion flask. Add 0.0557 g of the catalyst Se and 10 ml sulfuric Acid 98%. Suspending the sample by gently swirling the tube. Bring the digestion tube/flask and mixture into the digestion unit and into a heating block. Heat the mixture 370 °C until white fumes can be seen. The digestion is finished when the sample will be totally transparent. The sample is allowed to cool to room temperature and to diluted to 100 ml of water is added.

*Nitrogen determination in liquid digestate.* The Kjeldahl method was used to determine the nitrogen in liquid AD. Weighing approx. 4.0 g of the homogeneous sample. Place the sample into a digestion flask. Add 0.0557 g of the catalyst Se and 10 ml sulfuric Acid 98%. Carefully suspend the sample by gently swirling the tube. Bring the digestion tube/flask and mixture into the digestion unit and into a heating block. Heat the mixture 370 °C until white fumes can be seen. The digestion is finished when the sample will be totally transparent. The sample is allowed to cool to room temperature and to diluted to 100 ml of water is added.

*Organic carbon determination in solid AD.* For determining of organic carbon content in solid AD was used the method of dichromate oxidation. Mix thoroughly the dry digestate sample, weigh 0,0500 g ($\pm$0,0001g) and transfer to the Erlenmeyer 250ml flask. Place a mixture of 10ml potassium bichromate and sulphuric acid on the dry sample, cover with a crucible and leave to stand overnight at room temperature. The next day we pour 40ml of the mixture of potassium dichromate and sulphuric acid and heat in the thermostat at +160C for 30min. After cooling the flask, dilute its contents with distilled water into a 250 ml graduated flask. Mix the solution and leave to stand for 18-20 hours. The optical density is measured with an automatic spectrophotometer at a wavelength of 590 nm.

Calculation:

$$OC\% = A * 100\% / m$$

where

- m- weighed sample mass
- A- readings from a spectrophotometer.

*Organic carbon determination in liquid AD.* For determining of organic carbon content in liquid AD was used the method of dichromate oxidation. Mix thoroughly the dry digestate sample, weigh 1.0000-1.5000 g ($\pm$0,0001 g) of liquid AD and transfer to the Erlenmeyer 250 ml flask. The sample is dried in a thermostat at 80$\square$C in the flask until the digestate dries (depending on the thickness of the sample, it takes 2-3 hours). Place a mixture of 10ml potassium bichromate and sulphuric acid on the dry sample, cover with a crucible and leave to stand overnight at room temperature. The next day we pour 40ml of the mixture of potassium dichromate and sulphuric acid and heat in the thermostat at +160C for 30min. After cooling the flask, dilute its contents with distilled water into a 50 ml graduated flask. Mix the solution and leave to stand for 18-20 hours. The optical density is measured with an automatic spectrophotometer at a wavelength of 590 nm.

Calculation:

$$OC\% = A * 100\% / m$$

where

- m- weighed sample mass
- A- readings from a spectrophotometer.

*Elements content determination in solid AD.* Atomic absorptiometry method was used to determine the elements in AD. The total potassium (K), total magnesium (Mg), total sodium (Na), total copper (Cu), total iron (Fe), total zinc (Zn) contents were determined using the atomic absorption spectrometry measurement on Aanalyst 200 (Perkin Elmer, USA) after the wet digestion process with sulfuric acid.

*Elements content determination in liquid AD.* Atomic absorptiometry method was used to determine the elements in AD. The total potassium (TK), total magnesium (Mg), total sodium (Na), total copper (Cu), total iron (Fe), total zinc (Zn) contents were determined using the atomic absorption spectrometry measurement on Aanalyst 200 (Perkin Elmer, USA) after the wet digestion process with sulfuric acid.

*Crude ash determination in solid digestate (SD).* The method of weighing (furnace heating) was used to determine the crude ash content in AD. Weigh the dried (at 65°C) mass of digestates 1 gr. into the heated crucibles. Heat the furnace oven for 4 hours at 550 °C. We weigh and calculate the cooled crucibles:

Formula a) X (weight of crucible in grams containing sample after heating) - y (weight of empty crucible in grams) :1 (weight of sample in grams) x 100: dry matter = ash, % in dry matter. Analysis takes 8 hours of work. Dry samples are weighed with METTLER Toledo AG204 scales (max 210g) and liquid with OHAUS scales (max 500g). Analysis takes 8 hours of work. Dry samples are weighed with METTLER Toledo AG204 scales (max 210g) and liquid with OHAUS scales (max 500g).

*Crude ash determination in liquid digestate (LD).* The method of weighing (furnace heating) was used to determine the crude ash content in AD. Weigh in grams of liquid mass into heated crucibles. Heat the furnace oven for 4 hours at 550 °C. Weigh the heated crucibles and calculate (formula a). Analysis takes 8 hours of work. Dry samples are weighed with METTLER Toledo AG204 scales (max 210g) and liquid with OHAUS scales (max 500g).

*Total solids (TS) content determination in solid AD.* The method of weighing was used to determine TS.

*TS content determination in liquid AD.* The method of weighing was used to determine TS.

*Organic carbon (OC) determination in solid digestate.* Dichromate oxidation method was used to determine OC in solid AD.

*Organic carbon (OC) determination in liquid digestate.* Dichromate oxidation method was used to determine OC in liquid AD.

*Ammonium nitrogen (N-NH$_4$) in liquid digestate.* N-NH4 was determined by spectrophotometry at the wavelength of 550 nm with the cuvette test on the photometer LCK 302 (HACH Lange, Germany) using a standard procedure.

*Nitrate nitrogen (N-NO$_3$) in liquid digestate.* N-NO$_3$ was determined at the wavelength of 345 nm with the cuvette test LCK 339 on photometer DR3900 (HACH Lange, Germany) using a standard procedure.

*Development of NIRS mathematical equations for digestate analysis*

Digestates from two aggregate states obtained during separation were investigated: liquid and dry.

Combining various optical data scattering correction methods (SNV - standard normal variate, D - detrend, etc.), different mathematical treatment methods (various degrees of derivatives, derivative calculation intervals, initial smoothing intervals), a mathematical model was sought that best describes the relationship between optical and chemical data; this step is called calibration of equations.

During the project, about 120 thousand models of equations for liquid digestate (78 samples) were inspected (calibrated) to find out the best statistical relationship between spectral data and 21 digestate analytes (approximately 6,000 thousand equations per each analyte). Both, a complete set of spectral bands from 400 to 2498 nm (1050 independent variables) and excluding water-representative bands (1400-1440; 1900-1950) were used.

The development of calibration equations for dry separated digestates generated a total of approximately 58 thousand equations. 3,200 equations were generated for each of the eighteen analytes of dry digestate. In order to eliminate the negative effect of water on the calibration accuracy, the separated dry digestates were dried under laboratory conditions in an oven at 65 °C to constant weight and ground in a Retsch ZM 200 ultra-centrifugal mill. Both, a complete set of spectral bands from 400 nm to 2498 nm and only certain bands were used during calibration (Table 3 and Table 4). The equations that best reflect the relationship between the chemical composition of the digestate as well as its dry matter parameters and the spectral data are given in Table 1 and Table 3, indicating the key parameters for the suitability of the equations to describe the relationship.

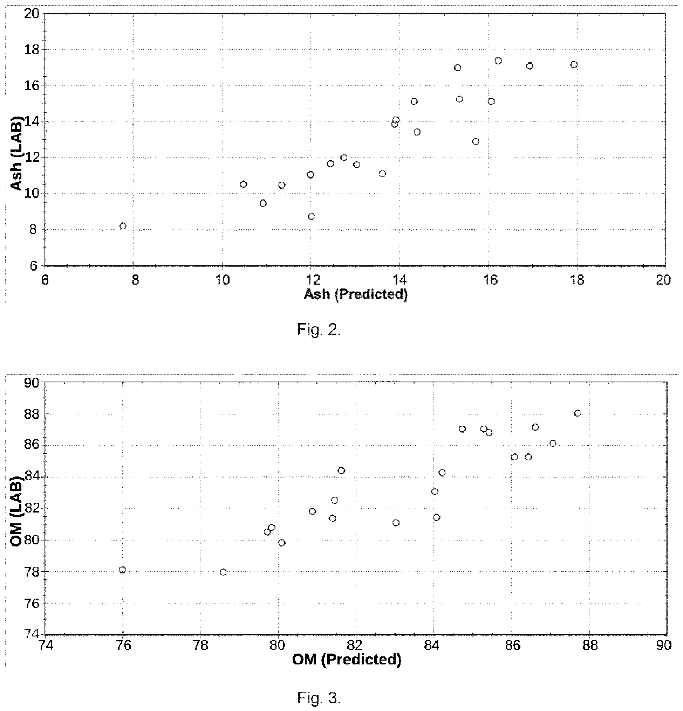

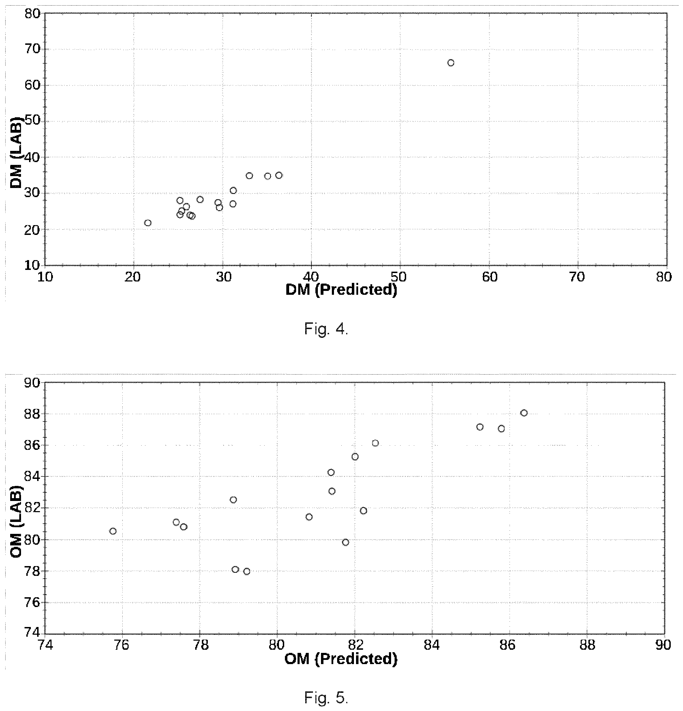

At this stage, the coefficients of determination (RSQ) of the calibrated equations for the different analytes of the liquid digestate were found to be high (0.7196-0.9828) and the coefficients of determination for the cross-validation (1-VR) ranged from 0.4736 to 0.8896 (Table 1). The worst statistics describe pH, sodium, nitrogen, iron, zinc, organic carbon and organic matter, and the best calibration results are for dry matter, mobile humic acids (MHA) and acid detergent liginin (ADL). Only 38 thousand out of more than 120,000 equations with 1-VR>= 0.4 were selected from the calibration results to test their ability to predict composition of the new samples. The 16 new samples were collected for this purpose. The aim was to detect the equations having substantial predictive properties during training, validation, and testing with new samples. The number of equations taken for testing varied greatly between digestate analytes, with some analytes (pH, OC, Na) not being tested at all due to low 1-VR, some tested only with a few dozen, and some with several thousand equations (total approx. 38 thousand). The best predictive equations with their parameters are presented in Table 2. Quantities of dry matter (RSQ = 0.943-0.903), organic matter (RSQ = 0.621-0.617), and ash content (RSQ = 0.621) are best predicted. Predictions from spectra for other analytes are less acceptable due to the large model errors (standard and bias) and low RSQ, most likely due to the heterogeneity of the digestate mass. Calibration graphs for ash in solid digestate (Fig. 2-3) and for OM and DM in liquid digestate (Fig. 4-5) are presented.

Table 1. Calibration results for equations describing the concentration of analytes in liquid digestate samples. N is the number of samples taken to develop equations (excluding outliers), SEC is the standard calibration error, RSQ is the calibration determination coefficient, SECV is the standard cross-evaluation error, 1-VR is the cross-evaluation determination coefficient. Match treatment: first number in the queue: function derivative; second number-gap between two data points; third number - function smoothing; fourth number- function smoothing 2.

| Analytel Units | N | Used spectral bands | Regression methods | Scatter methods | Math treatment | SE C | RSQ | SEC V | 1-VR |
|---|---|---|---|---|---|---|---|---|---|
| ADL % | 74 | All (400-2498) | ModPLS | WeightedMSC | 2,5,2,1 | 0,4 027 | 0,9828 | 1,574 6 | 0,7590 |
| ADF % | 74 | All except 1400-1440; 1900-1950 | ModPLS | WeightedMSC | 3,9,8,1 | 1,4 493 | 0,8401 | 2,474 5 | 0,5590 |
| NDF % | 74 | All (400-2498) | PLS | SNV_Detrend | 1,7,3,1 | 2,0 606 | 0,8863 | 4,017 2 | 0,5700 |
| Cu mg/kg | 73 | All except 1400-1440; 1900-1950 | ModPLS | None | 1,8,4,1 | 13, 194 3 | 0,9266 | 25,04 29 | 0,7331 |
| Fe mg/kg | 70 | All (400-2498) | PLS | WeightedMSC | 3,9,7,1 | 396 , 87 37 | 0,7453 | 534,9 263 | 0,5423 |
| Zn mg/kg | 71 | All except 1400-1440; 1900-1950 | PLS | Detrend | 1,2,2,1 | 77, 969 9 | 0,7196 | 99,42 79 | 0,5383 |
| MHS % | 73 | All except 1400-1440; 1900-1950 | ModPLS | WeightedMSC | 1,6,2,1 | 0,4 162 | 0,8939 | 0,690 8 | 0,7138 |
| MHA % | 73 | All except 1400-1440; 1900-1950 | PLS | StandardMSC | 1,6,3,1 | 0,2 121 | 0,9253 | 0,356 0 | 0,7866 |
| MFA % | 74 | All except 1400-1440; 1900-1950 | PLS | SNV | 1,4,3,1 | 0,2 023 | 0,9454 | 0,457 0 | 0,7376 |
| C/N | 71 | All (400-2498) | ModPLS | SNV_Detrend | 1,7,2,1 | 2,6 315 | 0,8744 | 5,272 4 | 0,5154 |
| OC % | 75 | All except 1400-1440; 1900-1950 | ModPLS | None | 3,7,3,1 | 2,1 572 | 0,8367 | 3,889 9 | 0,4736 |
| OM % | 73 | All except 1400-1440; 1900-1950 | ModPLS | Detrend | 1,2,2,1 | 1,4 487 | 0,8916 | 2,874 1 | 0,5677 |
| Na % | 70 | All (400-2498) | ModPLS | StandardMSC | 1,4,3,1 | 0,0 699 | 0,8692 | 0,140 9 | 0,4765 |
| Mg % | 71 | All (400-2498) | ModPLS | None | 1,3,3,1 | 0,0 792 | 0,9059 | 0,145 5 | 0,6829 |
| Ca % | 71 | All except 1400-1440; 1900-1950 | PLS | SNV_Detrend | 1,5,3,1 | 0,2 665 | 0,9048 | 0,510 4 | 0,6582 |
| K % | 69 | All (400-2498) | PLS | WeightedMSC | 1,6,4,1 | 0,0 941 | 0,8704 | 0,151 5 | 0,6629 |

(continued)

| Analytel Units | N | Used spectral bands | Regression methods | Scatter methods | Math treatment | SE C | RSQ | SEC V | 1-VR |
|---|---|---|---|---|---|---|---|---|---|
| P % | 71 | All except 1400-1440; 1900-1950 | PLS | StandardMSC | 1,6,2,1 | 0,1474 | 0,9286 | 0,3003 | 0,7023 |
| N % | 68 | All (400-2498) | PLS | StandardMSC | 3,8,7,1 | 0,0742 | 0,9516 | 0,2265 | 0,5470 |
| Ash % | 77 | All (400-2498) | ModPLS | WeightedMSC | 2,6,5,1 | 0,9890 | 0,9508 | 2,6647 | 0,6442 |
| DM % | 73 | All except 1400-1440; 1900-1950 | ModPLS | None | 2,7,2,1 | 0,8890 | 0,9700 | 1,6954 | 0,8976 |
| pH | 76 | All (400-2498) | ModPLS | WeightedMSC | 2,7,7,1 | 0,1325 | 0,8726 | 0,3122 | 0,3007 |

Table 2. Equations and their characteristics showing the best parameters for the prediction of liquid digestate composition. Presented only the equations which predict with a coefficient of determination > 0.4.

| Analyte / units | N | Used spectral bands | Regressi on methods | Scatter methods | Math treatmen t | SEP | RSQ | SECV | 1-VR |
|---|---|---|---|---|---|---|---|---|---|
| NDF % | 74 | All except 1400-1440; 1900 -1950 | ModPLS | None | 1,10,4,1 | 2,4870 | 0,501 | 4,2133 | 0,5242 |
| Fe mg/kg | 71 | All (400-2498) | ModPLS | SNV_Det rend | 1,6,2,1 | 1031,980 | 0,577 | 584,3304 | 0,4932 |
| Zn mg/kg | 74 | All except 1400-1440; 1900 -1950 | PLS | Weighted MSC | 2,4,3,1 | 107,0480 | 0,464 | 116,0163 | 0,4969 |
| MHS % | 76 | All except 1400-1440; 1900 -1950 | PCR | Detrend | 1,10,7,1 | 2,4780 | 0,509 | 0,9924 | 0,4863 |
| MFA % | 74 | All (400-2498) | ModPLS | Weighted MSC | 1,3,2,1 | 1,3210 | 0,436 | 0,5493 | 0,5521 |
| OM % | 76 | All (400-2498) | ModPLS | None | 3,24,19,1 | 2,6060 | 0,628 | 2,9711 | 0,6122 |
| Mg % | 73 | All (400-2498) | ModPLS | None | 2,24,23,1 | 0,2010 | 0,528 | 0,1666 | 0,6181 |
| K % | 74 | All (400-2498) | PLS | Weighted MSC | 2,7,3,1 | 0,3960 | 0,522 | 0,2010 | 0,4898 |
| Ash % | 78 | All (400-2498) | ModPLS | None | 2,19,4,1 | 2,3420 | 0,621 | 2,9010 | 0,5810 |
| DM % | 73 | All except 1400-1440; 1900 -1950 | ModPLS | Detrend | 1,6,3,1 | 3,2190 | 0,943 | 1,6726 | 0,9004 |

In the case of dry digestate, the most accurate was the *Modified PLS* regression method. The coefficients of determination (RSQ) of the calibration equations for all eighteen analytes ranged between 0.5664 for organic carbon and 0.9915 for magnesium (a total of about 58,000 model equations were calibrated). The coefficients of determination for cross-validation (1-VR) had a minimum value of 0.2462 for the determination of the contents of organic carbon and a maximum value of 0.8945 for ash (Table 3). The worst calibration results were shown by the organic carbon, pH, ADF, and iron equations, while the best results were obtained by the ash, copper, ADL, calcium, and JFR equations.

As in the case of liquid digestates, the most promising equations that showed the best results during calibration were tested with new samples (not included in the calibration of equations). 21 samples were used for this purpose. The equations showing their best performance and their characteristics are presented in Table 4. The highest coefficient of prediction (RSQ) - 0.813, was demonstrated by the ash calibration equation, its standard error of prediction (SEP) was 1.375. Slightly worse results were shown for organic carbon with RSQ of 0.807 and SEP of 1.404. The calibration equations for magnesium and potassium, with RSQ 0.788 and 0.724, SEP 0.145 and 0.246, respectively, also showed sufficiently good predictive properties. Other analytes of separated dry digestate are predicted with less accuracy. Improving the predictability of current equations requires constant updating and verification. It is standard NIRS practice to check whether new samples have been included into the calibration data sets, if not, this is done followed by recalibration of the equations. This procedure improves the accuracy of the prediction, and the equation adapts to changing samples.

Table 3. Calibration results for analytes describing the concentration of dry digestate (dried to constant weight at 65 °C). N is the number of samples taken to construct the equation (excluding exceptions), SEC is the standard calibration error, RSQ is the calibration determination factor, SECV is the standard cross-evaluation error, 1-VR is the cross-evaluation determination factor.

| Analyte / units | N | Used spectral bands | Regressio n methods | Scatter methods | Math treatmen t | SEC | RSQ | SECV | 1-VR |
|---|---|---|---|---|---|---|---|---|---|
| ADL % | 66 | 464-2418 | ModPLS | SNV_Detr end | 4,13,13,1 | 0,8226 | 0,9441 | 1,2563 | 0,8676 |
| ADF % | 67 | 408-2492 | ModPLS | Standard MSC | 4,13,8,1 | 1,7137 | 0,8078 | 3,0254 | 0,3919 |
| NDF % | 65 | 508-2492 | ModPLS | Standard MSC | 3,6,3,1 | 2,3326 | 0,8490 | 2,7537 | 0,7862 |
| Cu mg/kg | 66 | 408-2162 | ModPLS | SNV | 3,11,6,1 | 7,5804 | 0,9762 | 17,7419 | 0,8679 |
| Fe mg/kg | 67 | 408-2492 | ModPLS | None | 4,15,6,1 | 680,4988 | 0,5428 | 741,929 3 | 0,4483 |
| Zn mg/kg | 67 | 408-2492 | ModPLS | SNV | 1,6,1,1 | 70,8876 | 0,8323 | 82,6171 | 0,7687 |
| MHA % | 65 | All (400-2498) | ModPLS | Detrend | 0,30,30,1 | 0,3050 | 0,8748 | 0,3643 | 0,8186 |
| MFA % | 65 | 408-2492 | ModPLS | Standard MSC | 4,6,6,1 | 0,1804 | 0,9631 | 0,4699 | 0,7457 |
| OC% | 67 | 408-2492 | ModPLS | SNV_Detr end | 1,6,2,1 | 3,5304 | 0,5664 | 4,6197 | 0,2462 |
| OM % | 67 | 408-2492 | ModPLS | Standard MSC | 4,25,19,1 | 2,1097 | 0,8300 | 2,4389 | 0,7693 |
| Na % | 66 | 408-614; 678-1008; 1322-2498 | ModPLS | SNV | 2,33,1,1 | 0,1294 | 0,6756 | 0,1537 | 0,5353 |
| Mg % | 67 | 708-2492 | ModPLS | SNV_Detr end | 2,2,2,1 | 0,0282 | 0,9915 | 0,1777 | 0,6569 |
| Ca % | 64 | 408-2492 | ModPLS | None | 2,6,4,1 | 0,1749 | 0,9644 | 0,3680 | 0,8401 |
| K% | 66 | 408-2492 | ModPLS | SNV | 2,13,4,1 | 0,0804 | 0,9128 | 0,1756 | 0,5775 |
| P % | 62 | 550-610; 1160-1560; 1750-2060; 2130-2220 | ModPLS | SNV_Detr end | 3,15,7,1 | 0,2401 | 0,8292 | 0,2899 | 0,7468 |
| N % | 64 | 408-2492 | ModPLS | Standard MSC | 4,9,6,1 | 0,1904 | 0,7844 | 0,2836 | 0,5141 |
| Ash% | 65 | 408-2492 | ModPLS | Standard MSC | 2,17,4,1 | 1,0617 | 0,9433 | 1,4373 | 0,8945 |
| pH | 67 | 408-2492 | ModPLS | SNV | 1,5,4,1 | 0,2337 | 0,6226 | 0,3026 | 0,3575 |

Table 4. Equations and their characteristics showing the best parameters for the prediction of dry digestate composition. Presented only the equations which predict with a coefficient of determination > 0.4.

| Analyte / Units | N | Used spectral bands | Regression methods | Scatter method s | Math treatment | SEP | RSQ | SECV | 1-VR |
|---|---|---|---|---|---|---|---|---|---|
| ADL% | 67 | 408-2492 | ModPLS | None | 4,13,4,1 | 1,5357 | 0,8070 | 1,292 | 0,670 |
| NDF % | 65 | 508-2492 | ModPLS | Standard MSC | 3,6,3,1 | 2,9037 | 0,7623 | 2,069 | 0,657 |
| Cu mg/kg | 67 | 708-2492 | ModPLS | SNV | 3,11,6,1 | 23,0362 | 0,7739 | 18,855 | 0,711 |
| Fe mg/kg | 67 | 408-2492 | ModPLS | None | 4,15,6,1 | 769,4552 | 0,4066 | 942,785 | 0,530 |
| MHA % | 66 | 408-2492 | ModPLS | Detrend | 0,22,20,1 | 0,4207 | 0,7598 | 0,796 | 0,480 |
| OM % | 67 | 408-2492 | ModPLS | Standard MSC | 4,25,19,1 | 2,5984 | 0,7382 | 1,404 | 0,807 |
| Mg % | 67 | 408-2492 | ModPLS | SNV_De trend | 2,4,1,1 | 0,2304 | 0,4229 | 0,145 | 0,788 |
| K % | 66 | 408-2492 | ModPLS | SNV | 2,13,9,1 | 0,1822 | 0,5450 | 0,246 | 0,724 |
| Ash% | 67 | 958-2492 | ModPLS | Standard MSC | 2,17,4,1 | 2,3532 | 0,7259 | 1,375 | 0,813 |

The number of digestate analytes that can be precisely predicted by the application of the equations, detected by the described method, is different for liquid and dry separated digestate samples. Equations developed for the liquid digestate, can successfully predict 3 analytes: with high precision (RSQ>0.9) - 1 (dry matter), medium precision (RSQ>0.6) - 2 (organic matter and ash content). In case of dry digestate, prediction of relatively high precision (RSQ>0.8) applies to 2 analytes (ash content and organic carbon), medium precision (RSQ>0.7) - to 2 more analytes (magnesium and potassium). The total number of well predicted analytes in both liquid and dry digestate models is seven, three among them are predicted at high precision. By this method, the remaining analytes are determined with lower but sufficient for analysing precision.

NON-PATENT LITERATURE

1. Albrecht R., Joffre R., Gros R., Le Petit J., Terrom G., Périssol C. 2008. Efficiency of near-infrared reflectance spectroscopy to assess and predict the stage of transformation of organic matter in the composting process. Bioresourse Technology, 99: 448-55.

2. Albrecht R., Joffre R., Le Petit J., Terrom G., Perissol C. 2009. Calibration of Chemical and Biological Changes in Co composting of Biowastes Using Near-Infrared Spectroscopy. Environmental Science and Technology, 43: 804-811.

3. Albrecht R., Petit J., Terrom G., Périssol C. 2011. Comparison between UV spectroscopy and nirs to assess humification process during sewage sludge and green wastes co-composting. Bioresource Technology, 102: 4495-4500.

4. Bec K.B., Grabska J., Huck Ch. W. 2021. NIR spectroscopy of natural medicines supported by novel instrumentation and methods for data analysis and interpretation. Journal of Pharmaceutical and Biomedical Analysis 193: 113686.

5. Beccaccia A., Ferrer P., Ibáñez M. A., Estellés F., Rodriguez C., Moset V., Blas C., Calvet S., García-Rebollar P. 2015. Relationships among slurry characteristics and gaseous emissions at different types of commercial Spanish pig farms. Spanish Journal of Agricultural Research, 13.

6. Fujiwara T., Murakami K. 2007. Application of near infrared spectroscopy for estimating available nitrogen in poultry manure compost. Soil Science Plant Nutrition, 53:10.

7. Galvez-Sola L., Moral R., Moreno-Caselles J., Perez-Murcia M.D., Perez-Espinosa A., Bustamante M.A. 2009 a. Effectiveness of near infrared reflectance spectroscopy in the quick evaluation of nitrogen content in sewage sludge. Communications in Soil Science and Plant Analysis, 40:726-35.

8. Galvez-Sola L., Moral R., Perez-Murcia M.D., Perez-Espinosa A., Bustamante M.A., Martinez-Sabater E., Paredes C. 2010. The potential of near infrared reflectance spectroscopy (NIRS) for the estimation of agro-industrial compost quality. Science of the Total Environment, 408:1414-1421.

9. Galvez-Sola L., Morales J., Mayoral A.M., Paredes C, Bustamante M.A., Marhuenda-Egea F.C., Barber J. X., Moral R.I. 2013. Estimation of parameters in sewage sludge by near-infrared reflectance spectroscopy (NIRS) using several regression tools. Talanta 110: 81-88.

10. Givens, D., De Boever, J., Deaville, E. 1997. The principles, practices and some future applications of near infrared spectroscopy for predicting the nutritive value of foods for animals and humans. Nutrition Research Reviews, 10(1).

11. Huang G., Han L., Yang Z., Wang X. 2008. Evaluation of the nutrient metal content in Chinese animal manure compost using near infrared spectroscopy (NIRS). Bioresource Technology, 99: 8164-8169.

12. Mallet A., Charnier C., Latrille E., Bendoula R., Steyer J.-Ph., Roger J.-M. 2021. Unveiling non-linear water effects in near infrared spectroscopy: A study on organic wastes during drying using chemometrics. Waste Management, 122: 36-48.

13. Malley D.F., McClure C., Martin P.D., Buckley K., McCaughey W.P. 2005. Compositional analysis of cattle manure during composting using a field-portable near-infrared spectrometer. Communication Soil Science and Plant Analysis, 36:455-75.

14. Moral R., Galvez-Sola L., Moreno-Caselles J., Perez-Murcia M.D., Perez-Espinosa A., Paredes C. 2007. Can Near Infrared Reflectance Spectroscopy (NIRS) predict heavy metals in sewage sludge? In: Kungolos A., Aravossis K., Karagiannidis A., Samaras P., editors. First Conference on Environmental Management, Engineering, Planning and Economics, Skiathos island, Greece: 1683-8.

15. Murray, I. 2001. Theory and Principles of Near Infrared Spectroscopy, Article No. 1. In: 20 ans d'infrarouge au Centre de Rechzrches agronomiques: Journee d'Etude organisee a l'occasion du depart a la retraite de Mon- sieur Robert iston, Directeur du CRA, Gembloux, Belgium.

16. Near Infrared Reflectance Spectroscopy (NIRS): Analysis of Forage Quality. (1989) Editors: G.C. Marten J.S. Shenk F.E. Barton. / United States Department of Agriculture, Agricultural Research Service, Agriculture Handbook No. 643.

17. Ozaki Y., Huck C.W., Bec K.B. 2018. Near-IR spectroscopy and its applications, in: V.P. Gupta (Ed.), Molecular and Laser Spectroscopy. Advances and Applications, Elsevier, San Diego, CA, USA. 11-38.

18. Polak J., Sulkowski W.W., Bartoszek M., Papiez W. 2005. Spectroscopic studies of the progress of humification processes in humic acid extracted from sewage sludge. Molecular Structure, 744: 983-989.

19. Vergnoux A., Guiliano M., Le Dréau Y., Kister J., Dupuy N., Doumenq P. 2009. Monitoring of the evolution of an industrial compost and prediction of some compost properties by NIR spectroscopy. Science Total Environmental, 407:2390-403.

20. Xing L., Chen L.J, Han L.J. 2008. Rapid analysis of layer manure using near-infrared reflectance spectroscopy. Poultry Science, 87:1281-6.

21. Yang Z.L., Han L.J., Fan X. 2006. Rapidly estimating nutrient contents of fattening pig manure from floor scrapings by near infrared reflectance spectroscopy. Journal of Near Infrared Spectroscopy (14) 4: 261-268.

22. Zhang J., Sui Q., Li K., Chen M., Tong J., Li Q., Wei Y. 2017. Influence of natural zeolite and nitrification inhibitor on organics degradation and nitrogen transformation during sludge composting. Environmental Science and Pollution Research, 24: 9122.

23. Zhen L., Na Q., Yaning, L. 2016. Near-Infrared Spectroscopic Analysis of Green Waste Composting. Chemistry and Technology of Fuels and Oils, 52: 300-305.

**Claims**

1. A method for estimating the chemical composition of biogas-production anaerobic digestates AD by near-infrared spectroscopy NIRS, comprising steps at least of:

   • Providing weighted AD samples (2a, 2b);
   • performing NIRS analysis (4) on the provided weighted AD samples (2a, 2b), to obtain NIRS spectra (5) thereof;
   • applying for each obtained NIRS spectra (5) regression equations (6) comprising, at least, calibrated coefficients of determination RSQ between NIRS spectra and known chemical analytes, to estimate from the NIRS spectra (5) the concentration of said known chemical analytes in the chemical composition (7) of the weighted AD samples (2a, 2b).

2. The method according to claim 1, **characterized in that** said weighted AD samples (2a, 2b) are provided of any of the solid state (1a) and the liquid state (1b) wherein the AD samples of each state are provided being pre-processed:

   • the solid AD sample is dried and milled (2a), preferably, to less of 1 mm particles,
   • the liquid AD sample is encapsulated (2b), preferably, into a high-density-transparent polyethylene HDPE bag.

3. The method according to claims 1 and 2, **characterized in that** further it comprises a step of calibrating the regression equations and RSQ coefficients from a plurality of AD calibration samples, to establish the prediction of the AD chemical composition from the NIRS spectra, wherein at least

   • a plurality of AD calibration samples collected and scanned by NIRS, and
   • the chemical analytes in the chemical composition of the AD estimated by any known sufficiently accurate chemical analysis means, such as, Kjeldahl method, method of dichromate oxidation, method of atomic absorptiometry, method of weighing, method of spectrophotometry at particular wavelengths.

4. The method according to claims 1 to 3, **characterized in that** the chemical analytes for estimating their concentration in the solid-state AD samples (2a) are, at least, neutral detergent fibre (NDF) %, Fe %, Zn %, mobile humic substances (MHS) %, mobile fulvic acids (MFA) %, organic matter (OM) %, Mg %, K %, ash %, dry matter (DM) %.

5. The method according to claims 1 to 2, **characterized in that** the chemical analytes for estimating their concentration in the liquid-state AD samples (2b) are, at least, ADL %, NDF %, Cu %, Fe %, mobile humic acids (MHA) %,-OM %, Mg %, K %, ash %.

6. The method according to claims 1 to 5, **characterized in that** the target chemical analytes for estimating their concentration in the AD samples (2b) are selected as components, compositions, compounds or materials having a value as organic fertilizers, soil improvers.

7. The method according to claim 1 to 3, **characterized in that** the methods applied in and for setting-up the regression equations (6) are at least, partial-least-squares PLS and modified-partial-least-squares ModPLS.

8. The method according to claim 1, **characterized in that** the NIRS spectrum wavelengths are in the range from 780 nm to 2500 nm.

9. The method according to claims 1 to 6, characterized to be used for estimating the value of anaerobic digestates used as a bio-fertilizers, and the improvers of the soil properties.

Fig. 1

Fig. 2.

Fig. 3.

Fig. 4.

Fig. 5.

**EUROPEAN SEARCH REPORT**

Application Number

EP 22 17 8046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZENNARO BASTIEN ET AL: "Agronomic characterization of anaerobic digestates with near-infrared spectroscopy", JOURNAL OF ENVIRONMENTAL MANAGEMENT, vol. 317, 2 June 2022 (2022-06-02), page 115393, XP093000170, AMSTERDAM, NL ISSN: 0301-4797, DOI: 10.1016/j.jenvman.2022.115393 * the whole document * | 1-9 | INV. G01N21/359 |
| A | FINZI A ET AL: "Effects of measurement technique and sample preparation on NIR spectroscopy analysis of livestock slurry and digestates", BIOSYSTEMS ENGINEERING, ELSEVIER, AMSTERDAM, NL, vol. 134, 17 April 2015 (2015-04-17), pages 42-54, XP029230308, ISSN: 1537-5110, DOI: 10.1016/J.BIOSYSTEMSENG.2015.03.015 * abstract; page 43, left column, paragraphs 2 - 4 * | 1-9 | |
| | | | TECHNICAL FIELDS SEARCHED (IPC) |
| | | | G01N |
| A | PENG WEI ET AL: "Applications of near infrared spectroscopy and hyperspectral imaging techniques in anaerobic digestion of bio-wastes: A review", RENEWABLE AND SUSTAINABLE ENERGY REVIEWS, ELSEVIERS SCIENCE, NEW YORK, NY, US, vol. 165, 20 May 2022 (2022-05-20), XP087086897, ISSN: 1364-0321, DOI: 10.1016/J.RSER.2022.112608 [retrieved on 2022-05-20] * abstract; chapter 4.3 * | 1-9 | |

-/--

The present search report has been drawn up for all claims

| Place of search | Date of completion of the search | Examiner |
|---|---|---|
| Munich | 23 November 2022 | Flentje, Farida |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 22 17 8046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HUANG G ET AL: "Evaluation of the nutrient metal content in Chinese animal manure compost using near infrared spectroscopy (NIRS)", BIORESOURCE TECHNOLOGY, ELSEVIER, AMSTERDAM, NL, vol. 99, no. 17, 1 November 2008 (2008-11-01), pages 8164-8169, XP023182722, ISSN: 0960-8524, DOI: 10.1016/J.BIORTECH.2008.03.025 [retrieved on 2008-04-28] * abstract; chapters 2 and 3 * | 1-9 | |
| A,D | REMY ALBRECHT ET AL: "Comparison between UV spectroscopy and nirs to assess humification process during sewage sludge and green wastes co-composting", BIORESOURCE TECHNOLOGY, ELSEVIER, AMSTERDAM, NL, vol. 102, no. 6, 14 December 2010 (2010-12-14), pages 4495-4500, XP028361179, ISSN: 0960-8524, DOI: 10.1016/J.BIORTECH.2010.12.053 [retrieved on 2010-12-22] * abstract; chapters 2 and 3 * | 1-9 | |
| A | ERNESTO A RESTAINO ET AL: "Prediction of the Nutritive Value of Pasture Silage by Near Infrared Spectroscopy (NIRS)", CHILEAN JOURNAL OF AGRICULTURAL RESEARCH, vol. 69, no. 4, 1 January 2009 (2009-01-01), XP055300248, DOI: 10.4067/S0718-58392009000400011 * abstract * | 1-9 | TECHNICAL FIELDS SEARCHED (IPC) |

The present search report has been drawn up for all claims

| Place of search | Date of completion of the search | Examiner |
|---|---|---|
| Munich | 23 November 2022 | Flentje, Farida |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9678002 B2
- US 20050010374 A1
- WO 2014060687 A2
- EP 1199555 A
- WO 2004042375 A
- WO 2020099657 A

**Non-patent literature cited in the description**

- **ALBRECHT R. ; JOFFRE R. ; GROS R. ; LE PETIT J. ; TERROM G. ; PÉRISSOL C.** Efficiency of near-infrared reflectance spectroscopy to assess and predict the stage of transformation of organic matter in the composting process. *Bioresourse Technology,* 2008, vol. 99, 448-55
- **ALBRECHT R. ; JOFFRE R. ; LE PETIT J. ; TERROM G. ; PERISSOL C.** Calibration of Chemical and Biological Changes in Co composting of Biowastes Using Near-Infrared Spectroscopy. *Environmental Science and Technology,* 2009, vol. 43, 804-811
- **ALBRECHT R. ; PETIT J. ; TERROM G. ; PÉRISSOL C.** Comparison between UV spectroscopy and nirs to assess humification process during sewage sludge and green wastes co-composting. *Bioresource Technology,* 2011, vol. 102, 4495-4500
- **BEC K.B. ; GRABSKA J. ; HUCK CH. W.** NIR spectroscopy of natural medicines supported by novel instrumentation and methods for data analysis and interpretation. *Journal of Pharmaceutical and Biomedical Analysis,* 2021, vol. 193, 113686
- **BECCACCIA A. ; FERRER P. ; IBÁÑEZ M. A. ; ESTELLÉS F. ; RODRIGUEZ C. ; MOSET V. ; BLAS C. ; CALVET S. ; GARCÍA-REBOLLAR P.** Relationships among slurry characteristics and gaseous emissions at different types of commercial Spanish pig farms. *Spanish Journal of Agricultural Research,* 2015, vol. 13
- **FUJIWARA T. ; MURAKAMI K.** Application of near infrared spectroscopy for estimating available nitrogen in poultry manure compost. *Soil Science Plant Nutrition,* 2007, vol. 53, 10
- **GALVEZ-SOLA L. ; MORAL R. ; MORENO-CASELLES J. ; PEREZ-MURCIA M.D. ; PEREZ-ESPINOSA A. ; BUSTAMANTE M.A.** Effectiveness of near infrared reflectance spectroscopy in the quick evaluation of nitrogen content in sewage sludge. *Communications in Soil Science and Plant Analysis,* 2009, vol. 40, 726-35
- **GALVEZ-SOLA L. ; MORAL R. ; PEREZ-MURCIA M.D. ; PEREZ-ESPINOSA A. ; BUSTAMANTE M.A. ; MARTINEZ-SABATER E. ; PAREDES C.** The potential of near infrared reflectance spectroscopy (NIRS) for the estimation of agro-industrial compost quality. *Science of the Total Environment,* 2010, vol. 408, 1414-1421
- **GALVEZ-SOLA L. ; MORALES J. ; MAYORAL A.M. ; PAREDES C ; BUSTAMANTE M.A. ; MARHUENDA-EGEA F.C. ; BARBER J. X. ; MORAL R.I.** Estimation of parameters in sewage sludge by near-infrared reflectance spectroscopy (NIRS) using several regression tools. *Talanta,* 2013, vol. 110, 81-88
- **GIVENS, D. ; DE BOEVER, J. ; DEAVILLE, E.** The principles, practices and some future applications of near infrared spectroscopy for predicting the nutritive value of foods for animals and humans. *Nutrition Research Reviews,* 1997, vol. 10 (1
- **HUANG G. ; HAN L. ; YANG Z. ; WANG X.** Evaluation of the nutrient metal content in Chinese animal manure compost using near infrared spectroscopy (NIRS). *Bioresource Technology,* 2008, vol. 99, 8164-8169
- **MALLET A. ; CHARNIER C. ; LATRILLE E. ; BENDOULA R. ; STEYER J.-PH. ; ROGER J.-M.** Unveiling non-linear water effects in near infrared spectroscopy: A study on organic wastes during drying using chemometrics. *Waste Management,* 2021, vol. 122, 36-48
- **MALLEY D.F. ; MCCLURE C. ; MARTIN P.D. ; BUCKLEY K. ; MCCAUGHEY W.P.** Compositional analysis of cattle manure during composting using a field-portable near-infrared spectrometer. *Communication Soil Science and Plant Analysis,* 2005, vol. 36, 455-75

- Can Near Infrared Reflectance Spectroscopy (NIRS) predict heavy metals in sewage sludge?. **MORAL R. ; GALVEZ-SOLA L. ; MORENO-CASELLES J. ; PE-REZ-MURCIA M.D. ; PEREZ-ESPINOSA A. ; PAREDES C.** First Conference on Environmental Management, Engineering, Planning and Economics, Skiathos island, Greece. 2007, 1683-8
- Theory and Principles of Near Infrared Spectroscopy. **MURRAY, I.** 20 ans d'infrarouge au Centre de Rechzrches agronomiques: Journee d'Etude organisee a l'occasion du depart a la retraite. Directeur du CRA, 2001
- Near Infrared Reflectance Spectroscopy (NIRS): Analysis of Forage Quality. Agriculture Handbook. United States Department of Agriculture, Agricultural Research Service, 1989
- IR spectroscopy and its applications. **OZAKI Y. ; HUCK C.W. ; BEC K.B.** Molecular and Laser Spectroscopy. Advances and Applications. Elsevier, 2018, 11-38
- **POLAK J. ; SULKOWSKI W.W. ; BARTOSZEK M. ; PAPIEZ W.** Spectroscopic studies of the progress of humification processes in humic acid extracted from sewage sludge. *Molecular Structure,* 2005, vol. 744, 983-989
- **VERGNOUX A. ; GUILIANO M. ; LE DRÉAU Y. ; KISTER J. ; DUPUY N. ; DOUMENQ P.** Monitoring of the evolution of an industrial compost and prediction of some compost properties by NIR spectroscopy. *Science Total Environmental,* 2009, vol. 407, 2390-403
- **XING L. ; CHEN L.J ; HAN L.J.** Rapid analysis of layer manure using near-infrared reflectance spectroscopy. *Poultry Science,* 2008, vol. 87, 1281-6
- **YANG Z.L. ; HAN L.J. ; FAN X.** Rapidly estimating nutrient contents of fattening pig manure from floor scrapings by near infrared reflectance spectroscopy. *Journal of Near Infrared Spectroscopy,* 2006, vol. 4 (14), 261-268
- **ZHANG J. ; SUI Q. ; LI K. ; CHEN M. ; TONG J. ; LI Q. ; WEI Y.** Influence of natural zeolite and nitrification inhibitor on organics degradation and nitrogen transformation during sludge composting. *Environmental Science and Pollution Research,* 2017, vol. 24, 9122
- **ZHEN L. ; NA Q. ; YANING, L.** Near-Infrared Spectroscopic Analysis of Green Waste Composting. *Chemistry and Technology of Fuels and Oils,* 2016, vol. 52, 300-305